# EUROPEAN PATENT SPECIFICATION

(11) **EP 2 301 603 B1**
(45) Date of publication and mention of the grant of the patent: **20.03.2019**
(21) Application number: 09758109.4
(22) Date of filing: 02.06.2009
(51) Int. Cl.: A61M 5/145

(54) **SYRINGE PUMP**
SPRITZENPUMPE
POMPE À SERINGUE

(30) Priority: 03.06.2008 JP 2008146176
(43) Date of publication of application: 30.03.2011
(73) Proprietor: Terumo Kabushiki Kaisha, Tokyo 151-0072 (JP)
(72) Inventor: NAKANISHI, Masaru, Fujinomiya-shi Shizuoka 418-0015 (JP)
(74) Representative: Mewburn Ellis LLP
(86) International application number: PCT/JP2009/002480
(87) International publication number: WO 2009/147842

(56) References cited:
- EP-A2- 1 110 569
- WO-A1-01/08726
- DE-A1- 10 335 226
- JP-A- 9 122 237
- JP-A- 10 192 399
- JP-T- 2003 520 625
- US-A1- 2003 229 311

## Description

### TECHNICAL FIELD

The present invention relates to a syringe pump.

### BACKGROUND ART

Conventionally, syringe pumps are widely used for nutritional support and infusion of blood or a drug such as a chemotherapeutic agent or anesthetic drug for patients.

A syringe pump is generally designed to infuse a drug or the like into a patient by fixing the main body of a syringe filled with the drug, and in this state, causing a plunger portion to press a slider assembly to push out the drug from the syringe main body by a piston motion. Hence, flow rate control can accurately be done as compared to pumps of other infusion schemes.

Concerning the syringe pump, various proposals have been made so far. For example, Japanese PCT National Publication No. 9-506288 proposes a syringe pump which detects that the slider assembly has come into contact with the plunger portion. According to this syringe pump, the user can reliably perform the operation of moving the slider assembly to the contact position of the plunger portion at the start of infusion of a drug or the like. It is also possible to detect separation of the slider assembly from the plunger portion.

Japanese Patent Laid-Open No. 2004-73373 proposes a syringe pump which detects that the syringe main body is correctly fixed in the syringe pump. This syringe pump can prohibit infusion of a drug or the like from starting before correctly fixing the syringe pump.

Japanese PCT National Publication No. 2002-066102 proposes a syringe pump which detects the occlusion state of the infusion system including the infusion line when infusing a drug or the like. This syringe pump can properly detect the occlusion state of the infusion system.
WO 01/08726 A1 proposes a syringe plunger driver system capable of engaging different sized syringes.
EP 1 110569 A2 proposes a syringe pump in which it can be detected whether the flange of the thumb rest of a syringe is set on the slider assembly of the syringe pump regardless of whether the pump is in operation or not.
US 2003/229311 A1 proposes a syringe plunger driver system capable of capturing syringes of widely varying sizes comprising a pair of asymmetric plunger retainer arms pivotally mounted to a plunger driver.

As described above, various proposals have been made to monitor the state of the syringe pump. Using the detection results also enables to control the pressure the slider assembly is exerting on the plunger.

### SUMMARY OF INVENTION

### TECHNICAL PROBLEM

The slider assembly of the syringe pump is configured to grip the plunger portion by a pair of grip members in the back-and-forth direction.

For this reason, if a negative pressure acts in the syringe when the pressing force acting on the plunger portion is small, a gap may be formed between the slider assembly and the plunger portion.

As long as the plunger portion stands still in tight contact with the slider assembly at its control position without moving, the drug in the syringe is never infused regardless of the negative pressure acting in the syringe.

However, if the plunger portion moves by the distance corresponding to the gap due to the negative pressure acting in the syringe, the drug is infused by an amount corresponding to the moving amount. That is, the drug is infused by an amount that does not comply with the control of the slider assembly.

For this reason, to cause the syringe pump to implement infusion by an appropriate amount, the slider assembly preferably tightly holds the syringe plunger portion without forming a gap.

On the other hand, the diameter and thickness of the syringe plunger portion are determined by the volume of the syringe. Hence, to tightly hold the syringe plunger portion, the slider assembly needs to be adaptable to plunger portions with various diameters and thicknesses. In addition, the operation of causing the slider assembly to hold the plunger portion is preferably as simple as possible from the viewpoint of eliminating human error.

The present invention has been made in consideration of the above-described problem, and has as its object to provide a syringe pump capable of holding the plungers of various types of syringes in tight contact with the slider assembly by a simple operation.

### SOLUTION TO PROBLEM

According to a first aspect of the invention, there is provided a syringe pump according to claim 1. According to a second aspect of the invention, there is provided a syringe pump according to claim 5.

A syringe pump includes syringe fixing portions 2b, 2c, and 5 integrally formed on a pump main body 2 to fix a syringe main body of a syringe S filled with a drug, guide members 74 and 80 arranged on the pump main body so as to guide, along a longitudinal direction of the syringe, a main tube member 65 running from the pump main body along the longitudinal direction of the syringe and a sub tube member 66 rotatably provided in the main tube member, a mesh mechanism 81 provided at one end of the sub tube member to unmesh, by manually operating a clutch lever 61 biased to a standby position, the sub tube member which is driven by a motor and biased so as to mesh with a threaded shaft body 75 with a thread continually formed in the longitudinal direction, and a slider assembly 6 provided at the other end of each of the main tube member and the sub tube member and pivotally supporting a pair of grip members 62 and 63 which grip a syringe plunger SP upon operating the clutch lever, characterized in that the slider assembly comprises a grip moving mechanism which brings the syringe plunger into tight contact with the slider assembly by opening the pair of grip members upon manually operating the clutch lever, after setting the syringe plunger, closing the pair of grip members upon canceling the manual operation, and moving the pair of grip members toward the slider assembly.

The clutch lever is provided to be rotatable about the sub tube member and rotates a rotating member 107 fixed at the other end of the sub tube member into the unmeshed state upon the manual operation, the slider assembly comprises a slider main body 100 fixed at the other end of the main tube member, and the grip moving mechanism comprises a first shaft body 110 and a second shaft body 114 each of which fixes a corresponding one of the pair of grip members at one end and is provided to be rotatable in a pivotal support hole portion 100b or 100d formed in the slider main body and movable in the longitudinal direction of the pivotal support hole portion, a first sector gear 111 provided to be slidable in the longitudinal direction of the first shaft body and rotatable integrally, a second sector gear 115 provided to be slidable in the longitudinal direction of the second shaft body and rotatable integrally, a first intermediate gear 118 and a second intermediate gear 122 which are rotatably pivotally supported (117 and 120) by the slider main body between the first sector gear and the second sector gear and meshed with the first sector gear and the second sector gear, respectively, a torsion spring 121 provided on one of the first intermediate gear and the second intermediate gear to move and bias the pair of grip members to the close state, a protrusion 118g running from the first intermediate gear in a radial direction so as to come into contact with a second abutment surface 61g of the clutch lever upon the manual operation, an inclined member 113 provided to connect the other end of the first shaft body to the other end of the second shaft body and having an inclined surface 113a which comes into contact with a first abutment surface 61h formed on the clutch lever, and helical compression springs 112 and 116 provided between the inclined member and the first sector gear and the second sector gear so as to move and bias the pair of grip members toward the slider main body.

### ADVANTAGEOUS EFFECTS OF INVENTION

According to the present invention, it is possible to provide a syringe pump capable of holding the plungers of various types of syringes in tight contact with the slider assembly by a simple operation.

Other features and advantages will be apparent from the following description taken in conjunction with the accompanying drawings, in which like reference characters designate the same or similar parts throughout the figures thereof.

### BRIEF DESCRIPTION OF DRAWINGS

The accompanying drawings, which are incorporated in and constitute a part of the specification, illustrate embodiments of the invention and, together with the description, serve to explain the principles of the invention.
Fig. 1A is a plan view of a syringe type infusion device 1 (to be also referred to as the device 1 hereinafter);
Fig. 1B is a front view of the device 1;
Fig. 2 is a plan view of an operation panel 10 of the device 1;
Fig. 3A is a plan view of the device 1;
Fig. 3B is a rear view of the device 1;
Fig. 3C is a right side view of the device 1;
Fig. 4 is a perspective view showing an outer appearance of how devices 1 are fixed to mounting tools 300 fixed to an infusion stand 200;
Fig. 5 is a perspective view showing the outer appearance of a state after the devices 1 are fixed to the mounting tools 300 fixed to the infusion stand 200, together with syringes S;
Fig. 6A is a perspective view showing the outer appearance of a slider assembly 6;
Fig. 6B is a left side view of the slider assembly 6 whose grip members 62 and 63 grip a small-diameter syringe plunger SP;
Fig. 6C is a left side view of the slider assembly 6 whose grip members 62 and 63 grip a large-diameter syringe plunger SP;
Fig. 7A is a right side view showing a slider driving mechanism together with the slider assembly 6;
Fig. 7B is a left side view showing the slider driving mechanism together with the slider assembly 6;
Fig. 8 is a sectional view showing main part of the slider driving mechanism together with the slider assembly 6;
Fig. 9 is an exploded view showing the first-half assembled state of the slider assembly 6 based on isometric projection while setting the Z direction as one of three axes;
Fig. 10 is an exploded view showing the second-half assembled state of the slider assembly 6 based on isometric projection;
Fig. 11A is a front view of a clutch lever 61 after manual operation;
Fig. 11B is a front view of the clutch lever 61 after manual operation;
Fig. 12A is a skeleton diagram for explaining the operation of a grip moving mechanism;
Fig. 12B is a skeleton diagram for explaining the operation of the grip moving mechanism;
Fig. 12C is a skeleton diagram for explaining the operation of the grip moving mechanism;
Fig. 12D is a skeleton diagram for explaining the operation of the grip moving mechanism;
Fig. 13A is a front view of the syringe plunger SP gripped; and
Fig. 13B is a front view of the syringe plunger SP gripped.

### DESCRIPTION OF EMBODIMENTS

An embodiment of the present invention will now be described in detail with reference to the accompanying drawings as needed.

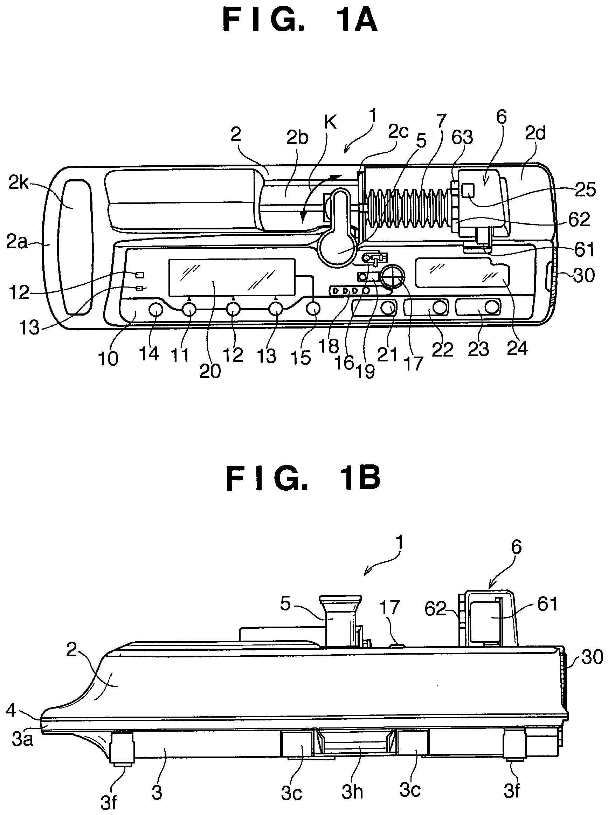

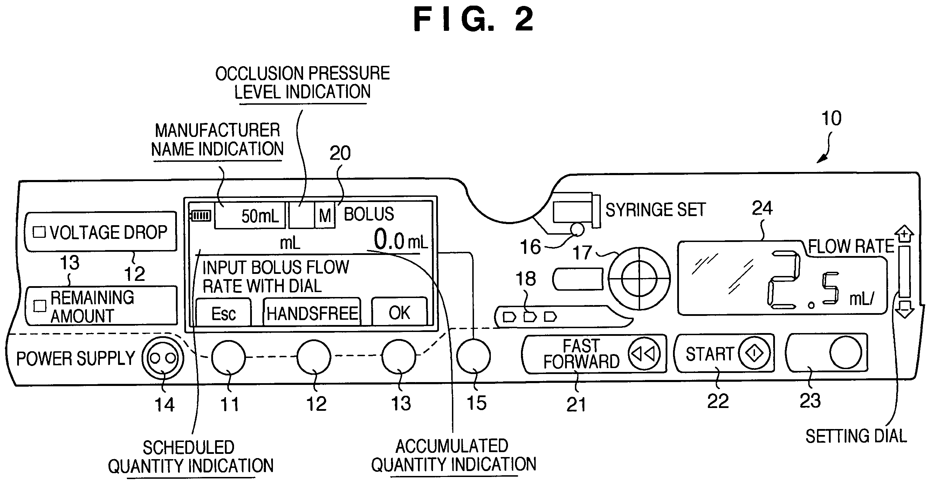

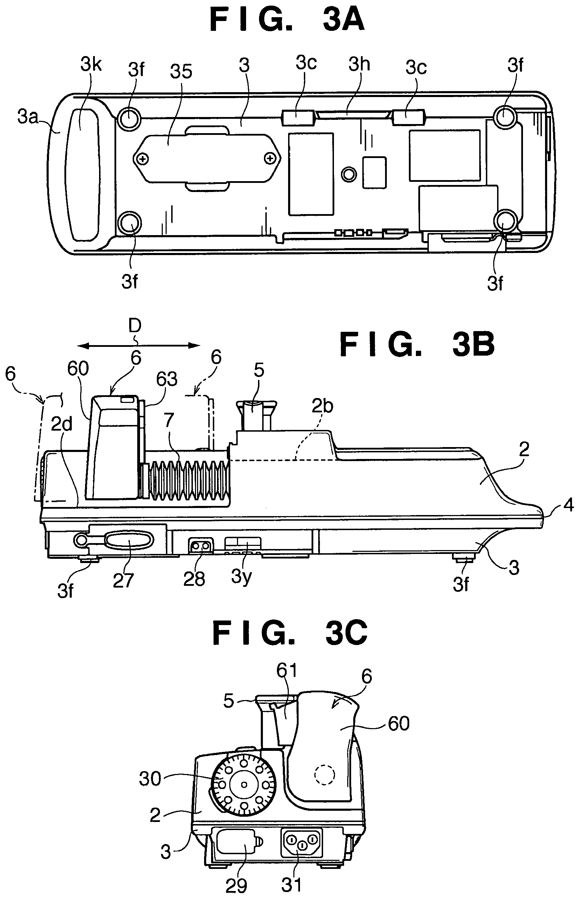

First of all, Fig. 1A is a plan view of a syringe pump 1 (to be also referred to as the device 1 hereinafter), and Fig. 1B is a front view of the device 1. Fig. 2 is a plan view of an operation panel 10 of the device 1. Fig. 3A is a plan view of the device 1. Fig. 3B is a rear view of the device 1. Fig. 3C is a right side view of the device 1.

Referring to Figs. 1A to 3C, the device 1 is also called a continuous microinfusion pump, which is used in an ICU, CCU, and NICU, and has as its main object to perform nutritional support, blood infusion, infusion of a drug such as a chemotherapeutic agent or anesthetic drug. Therefore, most part of the operation panel 10 is provided on the upper surface of the device 1 as shown in the accompanying drawings to easily display a flow rate and the like, thereby improving the operability.

The operation panel 10 is basically covered by an embossed resin sheet cover, and is provided with a drip proof design that can pass the splash proof test based on JISC0920. This provides the panel with high drip-proofness. Even if, therefore, a drug or the like drops on the cover due to, for example, carelessness, the drug can be easily wiped away and can be prohibited from entering the device 1.

Upper and lower covers 2 and 3 forming the main body of the syringe pump 1 each are integrally molded from a molding resin material having chemical resistance, and are configured to be able to be fixed to each other at the connecting surfaces of the covers 2 and 3 with screws (not shown) through a rubber seal 4 made of, for example, silicone elastomer. This arrangement completely prohibits a foreign substance such as a liquid from entering the pump.

Importance is placed on accurate infusion and an improvement in operability at the time of infusion. For this reason, accurate infusing operation control is implemented by microcomputer control, and an operation indicator 17 (see Figs. 1A and 1B) protruding upward at a position to provide high visibility from outside is placed at the position shown in Figs. 1A and 1B. The operation indicator 17 incorporates red and green light-emitting diodes, which turn on or blink in red or green or rotate/turn on to allow monitoring of an infusing operation state and a warning state even from a long distance, thereby reliably ensuring safety. In addition, the operation indicator 17 also incorporates a buzzer (not shown), that is, includes various types of safety-oriented warning functions.

Referring to Figs. 1A and 3A, in particular, the device 1 is compact and lightweight, and includes grip portions 2a and 3a which are integrally molded to extend from the left side surfaces of the upper and lower covers 2 and 3 through opening portions 2k and 3k. This allows the user to easily carry the device by gripping the grip portions 2a and 3a with hands by inserting his/her fingers in the opening portions 2k and 3k. The design has been made, in particular, to facilitate the concurrent use of a plurality of devices 1, as will be described later. The grip portions give accent in the industrial design.

Referring to Fig. 3C, in particular, the right side surface of the device 1 is provided with a setting dial 30 configured to quickly set a numerical value by being rotated in accordance with its rotational speed and rotating direction. In addition, a display unit 24 placed on the right side of the operation panel 10 can display a set value in accordance with the rotation of the setting dial 30. A set numerical value such as a flow rate can be easily changed with one action using the setting dial 30. The setting dial 30 can easily be removed especially for cleaning by rotating and moving it outside with his/her fingernail tip inserting in the gap between the dial and the upper cover.

Furthermore, the device 1 is designed to have a shape that allows multiple device use (concurrent use of many devices) (to be described later) without any unnecessary protrusions on the front and rear surfaces and the bottom surface so as to facilitate operation and allow further buildup. The specific dimensions of the device are: height H: 120 mm x width W: 360 mm x depth D: 117 mm. The weight of the device is as light as about 1.5 kg. The device uses three way power supplies including a commercial AC power supply, a built-in lithium-ion battery, and a DC 12 VA.

The charging time of the built-in battery is about 6 h (hours). The battery is covered by a lid member 35 (see Fig. 3A) at a bottom portion of the lower cover 3 to allow easy replacement of the battery from outside. The battery is connected to a connector to be detachable. The battery has a replacement lifetime of two years or more, and is provided with constant current/constant voltage charging control to prohibit overcharge. In addition, a monitoring element (to be described later) is connected to the built-in battery. This element monitors the capacity of the built-in battery, the voltage/current at the time of charging, the voltage/current at the time of discharging, and the like. This implements prohibition of overdischarge and overcharge. Using a heat-resistant lithium-ion battery, in particular, allows, if it is a new battery, the device to operate for 12 h (hours) until the generation of an alarm, and at least for about 12.5 h (hours) until shutdown.

A display unit 20 serves as the second display unit to display a scheduled quantity (mL), an accumulated quantity (mL), a gamma infusion rate (mg/kg/h), an occlusion history, a charge history, a message indicating the contents of operation, the information of an alarm, the manufacturer name of a set syringe, and the like. This display unit and the display unit 24 to display a flow rate (mL/h) are separately provided on the operation panel 10 at a predetermined distance of about 80 to 100 mm. The display unit 20 is preferably a liquid crystal display.

To fix the syringe in a stationary state, a mount base 2b and a recess portion 2c are integrally formed with the upper cover 2 by injection molding. A syringe flange portion integrally formed with the main body of the syringe is inserted in the recess portion 2c. A clamp 5 can be manually moved in the vertical direction, and is provided to freely rotate in the arrow K direction shown in Fig. 1A. Therefore, a clamp support for supporting the clamp 5 is also integrally formed.

A slider assembly 6 to be moved and driven between the respective positions indicated by the chain lines in Fig. 3B in both the arrow D directions reciprocally moves in the space above a step portion 2d of the upper cover 2 and is coupled and fixed to the slider assembly 6 (to be described later) through a main tube member 65 running from the pump main body along the longitudinal direction of the syringe and a sub tube member 66 rotatably provided in the main tube member. In the slider assembly 6, a slider cover 60 accommodates a clutch lever 61 protruding forward. Manually operating the clutch lever 61 toward the rear side allows a syringe plunger SP to be easily attached and detached.

The user places the syringe plunger of the syringe so as to bring it into contact with a side surface of the slider cover 60 of the slider assembly 6 after manually pressing the clutch lever 61 toward the rear side, and then releases the clutch lever 61. This makes a pair of grip members 62 and 63 automatically hold the syringe plunger from the back-and-forth direction. The slider assembly 6 is configured to have an important function for accurately feeding a drug stored in the syringe in accordance with a set/input value or reducing the internal syringe pressure by detachably holding syringe plungers SP with various different diameters in a stationary state and accurately driving each syringe plunger in the infusing direction or a direction opposite to the infusing direction.

A boot 7 for waterproofing covers the shaft. A lamp 109 is built in the upper surface of the slider assembly 6, which blinks or turns on when the grip members do not properly grip the syringe plunger and a sensor (not shown) detects this state.

Referring to Figs. 1A and 2 again, the operation panel 10 includes a power switch 14, an AC/DC lamp 13, and a battery lamp 12 to turn on upon a drop in voltage, which are disposed together on the left distal end of the panel. The large liquid crystal display unit 20 is disposed adjacent to these lamps. When the user fixes a syringe by using the clamp 5, the display unit 20 automatically measures the diameter of the syringe upon converting the movement amount of the clamp 5 in the vertical direction into an electrical signal, and automatically displays the volume of the fixed syringe in numerical form like 5 cc (mL), 10 cc (mL), 20 cc (mL), 30 cc (mL), 50 cc (mL), or 100 cc (mL).

In addition, a display unit 18, a remaining amount alarm lamp 19, and a clamp abnormality lamp 16 are disposed together almost in the middle of the device 1. The display unit 18 serves as the first display unit to sequentially display the pressures set/detected by the occlusion detection mechanism provided for the syringe pump 1 in three steps in different colors, including green, yellow, and orange. When the pressure reaches a set value (threshold), the display unit 18 displays it in red as an alarm. The clamp abnormality lamp 16 informs clamp fixation abnormality by turning on or blinking in red. The clamp abnormality lamp 16 has a shape imitating the shape of the syringe.

On the other hand, the display unit 24 incorporates preferably a 7-segment LED to display only a flow rate, and further includes an integration clear switch 15, a fast forward switch 21, a start switch 22, and a stop/mute switch 23. Note that the display unit 24 is configured to display a flow rate (mL/h) in orange when the flow rate is 0.9 mL/h or less, and in green when the flow rate is 1.0 mL/h or less in a size larger than that when the flow rate is 0.9 mL/h or less.

A sensor covered by a rubber cover (not shown) is provided near the recess portion 2c to detect that the main body of the syringe is set properly on the mount base 2b in the back-and-forth direction (X direction) in a stationary state.

Referring to Fig. 1B, a front recess portion 3h is formed in the front surface of the lower cover 3 at a position slightly shifted from the middle to the right in the pump main body. Abutment surfaces 3c are formed on the two sides of the front recess portion 3h. The front recess portion 3h and the abutment surfaces 3c serve to provide one lock portion to fix the device 1 to the mounting tool fixed to the infusion stand (to be described later). Four foot portions 3f are formed on the bottom surface to fix four rubber feet.

Referring to Fig. 3B, a rear recess portion 3y is formed in the middle of the rear surface of the lower cover 3. The rear recess portion 3y serves to provide the other lock portion to fix the device 1 to the mounting tool fixed to the infusing stand (to be described later). A contact 28 is provided on the left of the rear recess portion 3y to allow the device to receive power supply after it is set on the mounting tool. An external communication connector 27 with a drip proof cover is provided adjacent to the contact 28.

Referring to Fig. 3C, a 3P commercial power supply connector 31 and a nurse call connector 29 with a waterproof cover are provided on the right side surface of the lower cover 3.

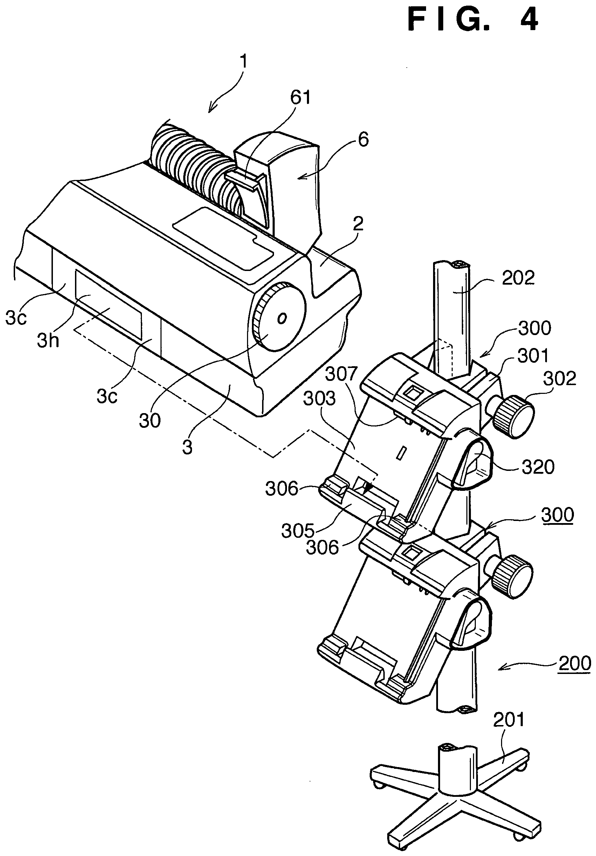

Fig. 4 is a perspective view showing an outer appearance of how the devices 1 are fixed to mounting tools 300 fixed to an infusion stand 200. In Fig. 4, the same reference numerals as those already described above denote the same components or parts, and a description thereof will not be repeated. As shown in Fig. 4, the device 1 is set on the mounting tool 300 while the front recess portion 3h and the abutment surfaces 3c on the front surface of the lower cover 3 described above face forward.

Each mounting tool 300 includes a clamp device 301 to fix the device at an arbitrary height position on a pole 202 fixed to a mobile stand 201 of the infusion stand 200 so as to stand upright. The clamp device 301 is provided with a fixing knob 302. Operating the fixing knob 302 can easily detach the mounting tool 300 and attach it to poles 202 having different diameters or a bed frame.

The mounting tool 300 has an inclined mount surface 303 inclined at an angle of about 60° relative to a horizontal plane and formed as shown in Fig. 4. A protrusion 305 which is fitted in the front recess portion 3h is formed below the inclined mount surface 303. In addition, abutment surfaces 306 which come into contact with the abutment surfaces 3c described above are formed on a lower portion of the inclined mount surface 303. A pawl member 307 which is fitted in the rear recess portion 3y is rotatably provided above the inclined mount surface 303. The device can be easily attached and detached by operating the pawl member 307 between a lock position and an unlock position with a lever 320 on a side surface. Fig. 4 shows a case in which the two mounting tools 300 are provided. Obviously, however, it is possible to provide one or any number of mounting tools other than two.

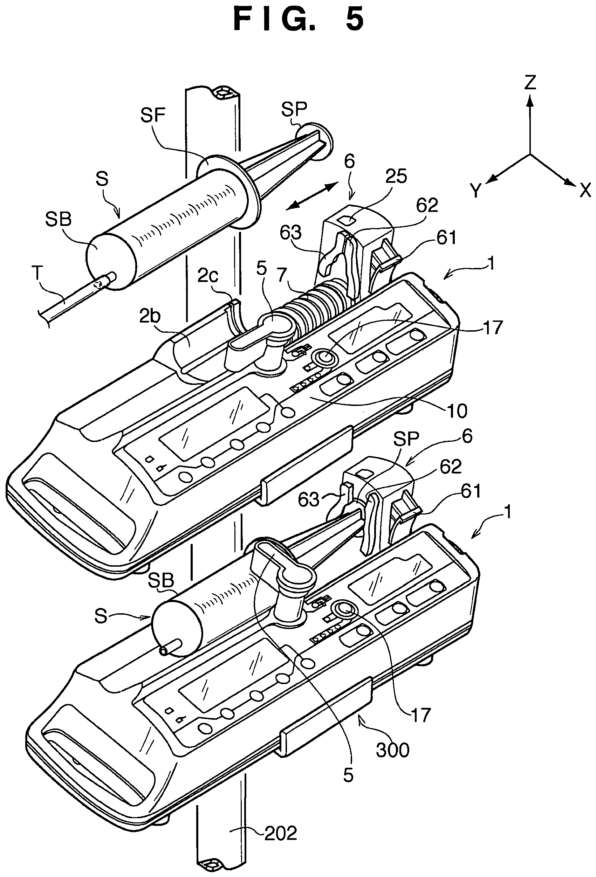

Fig. 5 is a perspective view showing an outer appearance of a state after the devices 1 are fixed to the mounting tools 300 fixed to the infusion stand 200, together with syringes S. In Fig. 5, the same reference numerals as those already described above denote the same components or parts, and a description thereof will not be repeated. As shown in Fig. 5, each device 1 is set on the corresponding mounting tool 300 while the front surfaces of the upper and lower covers 2 and 3 are positioned forward.

To set the syringe S, the user places a syringe main body SB on the mount base 2b after rotating the clamp 5 to the front side through 90°, and sets a syringe flange SF in the recess portion 2c. The user then pulls up the clamp 5 held spline-fitted on the vertical protrusion of the clamp support (not shown), and rotates the clamp 5 to the initial position. With this operation, the syringe can be reliably fixed with tension spring force (not shown), as shown in Fig. 5. If the syringe S is not reliably fixed by the clamp 5 and/or the syringe flange SF is not reliably set in the recess portion 2c, the clamp abnormality lamp 16 turns on or blinks in red to call attention of the user.

To make the slider assembly 6 grip the syringe plunger SP of the syringe S, the user presses the clutch lever 61 in the depth direction to open the pair of grip members 62 and 63, and releases the clutch lever 61 to move the pair of grip members 62 and 63 to the grip position. Detecting this gripped state interlockingly with a slider mechanism portion will detect that the syringe plunger SP is properly set on the slider assembly 6. For this purpose, a sensor actuator (to be described later) is provided between the pair of grip members 62 and 63. The sensor actuator is biased by the effect of a torsion spring such that one end always protrudes. The sensor actuator retracts to the rear side only when the syringe plunger SP is properly gripped. This makes it possible to detect a proper mounted state. The lamp 109 displays this state.

With the above operation, the syringe flange SF to be set in the recess portion 2c near the mount base 2b is properly set on the mount base in a stationary state in the lateral direction (Y direction). In addition, the syringe main body SB is properly set in the back-and-forth direction (X direction). A small-diameter arcuated groove portion is further integrally formed with the mount base 2b to allow a small-volume, small-diameter syringe to be held in a stationary state.

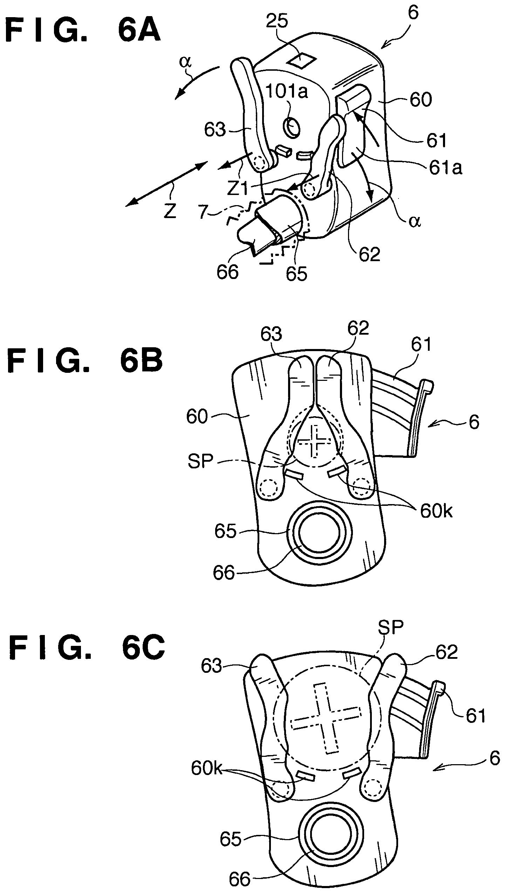

Fig. 6A is a perspective view showing the outer appearance of the slider assembly 6. Fig. 6B is a left side view of the slider assembly 6 whose grip members 62 and 63 grip a small-diameter syringe plunger SP. Fig. 6C is a left side view of the slider assembly 6 whose grip members 62 and 63 grip a large-diameter syringe plunger SP.

In Figs. 6A to 6C, the same reference numerals as those already described above denote the same components or parts, and a description thereof will not be repeated. The slider assembly 6 is provided at the distal ends of the main tube member 65 and the sub tube member 66 rotatably provided in the main tube member 65. The cover 60 is provided to cover the entire slider assembly 6. An operation surface 61a of the clutch lever 61 protrudes from the front surface. A transparent cover 25a is provided on the upper surface of the cover 60 to cover the lamp 109. The cover has a hole portion made in its left side surface at the middle portion between the pair of grip members from which an actuator portion 101a of the sensor actuator protrudes to the outside.

In the above-described arrangement, when the user manually presses the clutch lever 61 in the direction of an arrow, the slider assembly 6 can freely move in the direction of an arrow Z. In addition, the pair of grip members 62 and 63, which have been closed before the clutch lever operation, move in the direction of an arrow Z1 and then rotate by an angle α so as to open.

After that, the syringe plunger SP of the syringe fixed to the main body in the above-described manner is set between the pair of grip members. When the user releases the clutch lever, the gripped state shown in Fig. 6B or 6C is attained. Referring to Fig. 6C, molded portions 60k formed on the side surface of the cover 60 serve as a guide for the syringe plunger SP having the maximum diameter. Actually, the pair of grip members on the left and right sides automatically align and grip the syringe plunger.

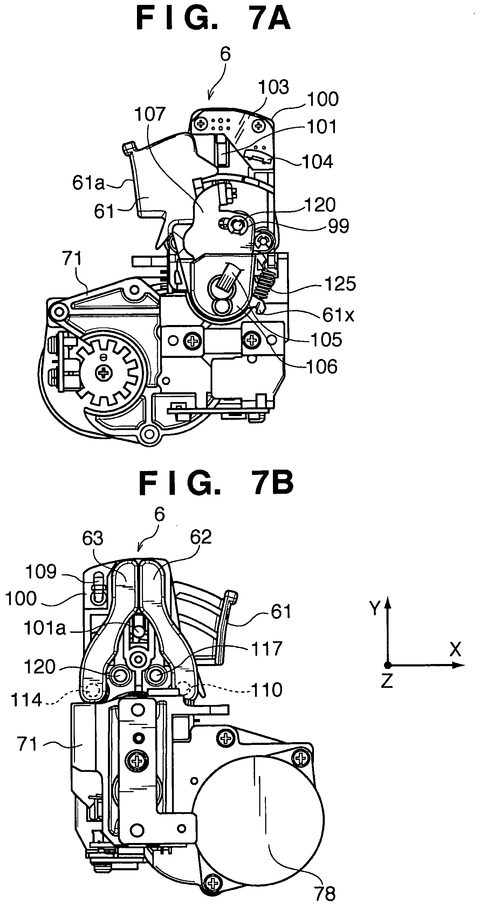

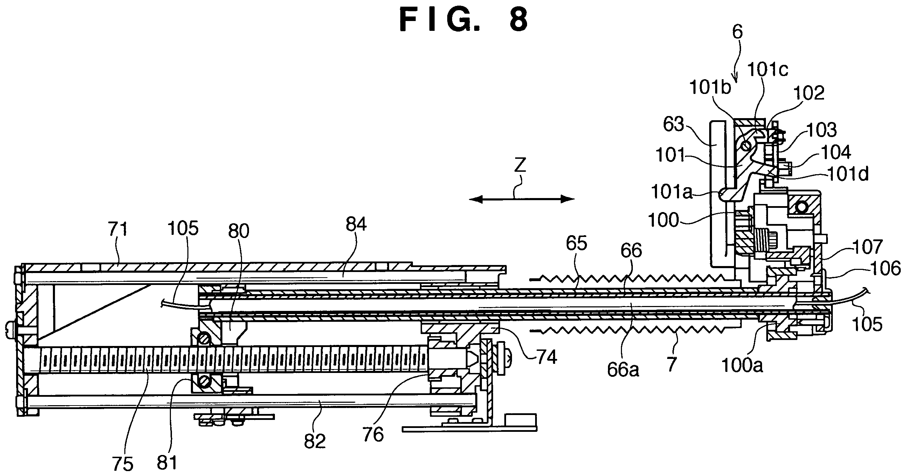

Fig. 7A is a right side view showing a slider driving mechanism together with the slider assembly 6. Fig. 7B is a left side view. Fig. 8 is a sectional view showing main part of the slider driving mechanism together with the slider assembly 6.

An example of the arrangement of a grip moving mechanism will be described below with reference to Figs. 7A, 7B, and 8. Needless to say, the grip moving mechanism can have any arrangement capable of opening the pair of grip members upon manually operating the clutch lever and then closing them upon releasing the manual operation, and subsequently bringing the syringe plunger into tight contact with the slider assembly 6 by moving the pair of grip members to the side of the slider assembly 6.

In Figs. 7A, 7B, and 8, the same reference numerals as those already described above denote the same components or parts, and a description thereof will not be repeated. The clutch lever 61 has a rotating member 107 which is provided to rotate about the sub tube member 66 and fixed at the other end of the sub tube member. The manual operation of the clutch lever 61 makes a half nut 81 fixed at one end of the sub tube member rotate into an unmeshed state. As a result, the slider assembly 6 can move in the direction of the arrow Z.

On the other hand, a slider main body 100 made of a resin is fixed to the other end of the main tube member 65. The pair of grip members are provided on the slider main body 100 so as to freely rotate and translate. The main tube member 65 and the sub tube member 66 are pivotally supported by guide members 74 and 80 arranged in the pump main body so as to translate. For this purpose, parallel shaft bodies 82 and 84 each having two ends fixed to a base portion 71 hold the guide members 74 and 80 not to rotate. The base portion 71 pivotally supports a threaded shaft body 75 on which a gear 76 meshed with a gear train to be driven by a stepping motor 78 is fixed.

A wiring line 105 connected to a light-shielded sensor 102 is designed to pass through the hollow portion of a sub tube member 66a and exit to the outside. A sensor actuator 101 having a pivotal support portion 101b pivotally supported by the slider main body 100 is provided so as to make an actuator 101c shield the light-shielded sensor 102 or rotate counterclockwise against a torsion spring (not shown) and cancel the shielding upon coming into contact with the syringe plunger as described above. The light-shielded sensor 102 is mounted on a sensor substrate 103 an connected to the wiring line 105 via a connector 104.

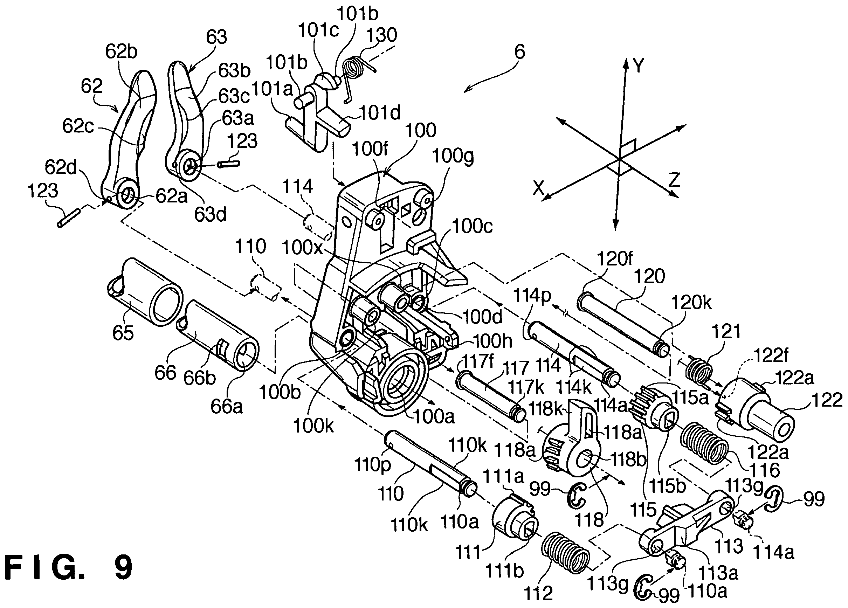

Fig. 9 is an exploded view showing the first-half assembled state of the slider assembly 6 based on isometric projection while setting the Z direction as one of three axes. In Fig. 9, the same reference numerals as those already described above denote the same components or parts, and a description thereof will not be repeated. The distal end of the main tube member 65 is inserted in a hole portion 100a of the slider main body 100 of made of a resin, and then fixed to integrate the main tube member 65 and the slider main body 100. The sub tube member 66 inserted in the hollow portion of the main tube member 65 passes through the hole portion 100a in the direction of an arrow. The rotating member 107 is fixed in left and right notches 66b.

The slider main body 100 forms a total of four pivotal support hole portions 100b, 100c, 100d, and 100k, as shown in Fig. 9. The pivotal support hole portions 100b and 100d receive a first shaft body 110 and a second shaft body 114, respectively, which are made of stainless steel. Pinhole portions 110p and 114p extending through in the radial direction, flat surfaces 110k and 114k formed in bilateral symmetry, small-diameter portions running from the step portions, and groove portions 110a and 114a formed on the small-diameter portions are machined on the shaft bodies 110 and 114, respectively, as shown in Fig. 9.

When the first shaft body 110 and the second shaft body 114 are inserted in the pivotal support hole portions up to the positions indicated by broken lines, closed-end hole portions 62a and 63a are set on the shaft bodies, and press-fit pins 123 are pressed into horizontal hole portions 62d and 63d and further inserted in the pinhole portions to fix the pair of grip members 62 and 63 which grip the syringe plunger SP upon operating the clutch lever.

The grip members 62 and 63 are formed from a predetermined resin material by injection molding, and have molded portions 62c and 63c, respectively, which are integrally formed as inclined surfaces so as to cause the edges of running portions 62b and 63b to grip the edge of the syringe plunger. A first sector gear 111 made of a resin is set to be slidable in the longitudinal direction of the first shaft body 110 and rotate integrally with it. A second sector gear 115 made of a resin is set to be slidable in the longitudinal direction of the second shaft body 114 and rotate integrally with it. For this purpose, the sector gears 111 and 115 have insertion hole portions 111b and 115b which are almost oval and are fitted on the flat surfaces 110k and 114k. Sector gear portions 111a and 115a are formed on the peripheral outside surfaces of the sector gears 111 and 115, as shown in Fig. 9. The internal diameter portions of helical compression springs 112 and 116 are set on the small-diameter portions of the sector gears 111 and 115, as shown in Fig. 9, so as to always move and bias the pair of grip members 62 and 63 to the side of the slider main body 100.

Next, an inclined member 113 made of a resin and having an inclined surface 113a and hole portions 113g formed as shown in Fig. 9 is assembled by fitting snap rings 99 on the groove portions 110a and 114a formed on the small-diameter portions of the first shaft body 110 and the second shaft body 114, which are indicated by broken lines. This allows the pair of grip members 62 and 63 to rotate and move in the longitudinal direction in the pivotal support hole portions.

A shaft body 117 made of stainless steel is inserted in the pivotal support hole portion 100k of the slider main body 100 from the rear surface side until its further movement is regulated by a flange portion 117f. A first intermediate gear 118 having sector gear portions 118a formed in bilateral symmetry, one of which meshes with the first sector gear 111, is fitted on the shaft body 117 so as to freely rotate about it, and set by fitting the snap ring 99 on a groove portion 117k. A protrusion 118k running in the radial direction as shown in Fig. 9 is integrally formed on the first intermediate gear 118 so as to abut against a second abutment surface 61g of the clutch lever first, as will be described later.

A second intermediate gear 122 that meshes with the second sector gear 115 and the first intermediate gear 118 has sector gear portions 122a formed in bilateral symmetry. A shaft body 120 made of stainless steel is inserted in the pivotal support hole portion 100c of the slider main body 100 from the rear surface side until its further movement is regulated by a flange portion 120f. The second intermediate gear 122 is fitted on the shaft body 120 so as to freely rotate about it, and set by fitting the snap ring 99 on a groove portion 120k.

A hole portion 122f is formed on the side surface of the second intermediate gear 122. A torsion spring 121 wound in advance to move and bias the grip members to the close state is set as shown in Fig. 9. The hole portion 122f holds one of the action end of the torsion spring 121, and the other action end is set in a hole portion 100x of the slider main body 100. As the torsion spring 121 returns to the natural state, the sector gears 111 and 115 rotate to close the grip members.

The sensor actuator 101 is set in a space portion 100f of the slider main body 100 after setting a torsion spring 130 on the pivotal support portion 101b. The sensor substrate is screwed to threaded holes 100g of the slider main body 100.

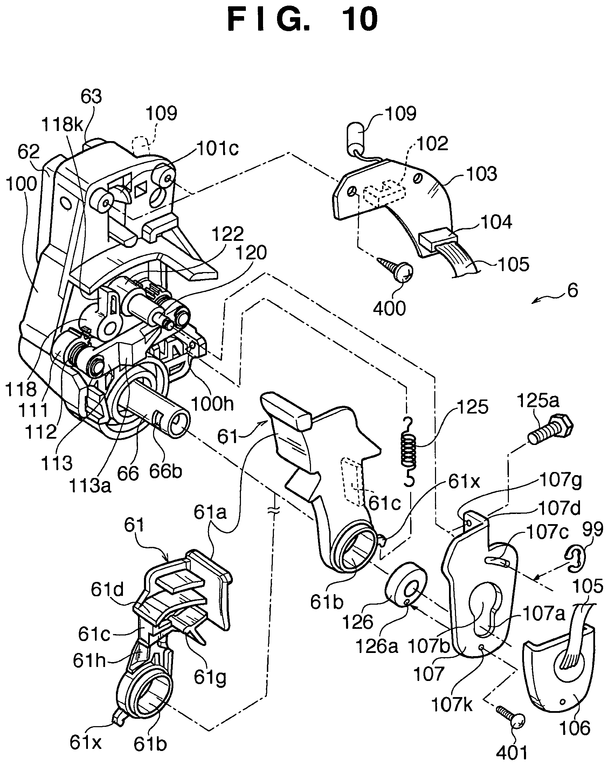

Fig. 10 is an exploded view showing the second-half assembled state of the slider assembly 6 based on isometric projection. In Fig. 10, the same reference numerals as those already described above denote the same components or parts, and a description thereof will not be repeated. With the assembly operation based on Fig. 9, the sub tube member 66 protrudes to the outside, as shown in Fig. 10. The actuator 101c of the sensor actuator 101 protrudes to the outside. The pair of grip members 62 and 63 are in contact with the slider main body 100. The protrusion 118k running from the first intermediate gear 118 in the radial direction is located above. The inclined surface 113a of the inclined member 113 is exposed to the outside.

In this state, the sensor substrate 103 on which the LED lamp 109 is fixed using machine screws 400 so that the actuator 101c shields the sensor 102, and the lamp 109 is fixed at the position indicated by a broken line.

The clutch lever 61 is made of a resin. A hole portion 61b to be fitted on the sub tube member 66 while placing the operation surface 61a on the front side, a hook portion 61x which supports one end of a helical extension spring 125, and a flat surface 61c are integrally formed on the clutch lever 61. The other end of the helical extension spring 125 is supported by a hook portion 100h of the slider main body 100 so that the clutch lever 61 is always rotatably and pivotally supported counterclockwise. A second abutment surface 61g which comes into contact with the protrusion 118k, the flat surface 61c, and a first abutment surface 61h formed at a position where it comes into contact with the inclined surface 113a of the inclined member 113 earlier than the second abutment surface 61g are also integrally formed on the clutch lever 61 whose opposite side is also illustrated in Fig. 10.

After the clutch lever 61 is set by inserting the sub tube member 66, an annular member 126 is set. The rotating member 107 formed from a metal plate is fixed to the sub tube member 66. The second shaft body 120 is inserted in a long groove portion 107c and fixed by fitting the snap ring 99 on the groove portion. The rotating member 107 has a bent portion with a 3-mm threaded hole 107g. A nut and a screw 125a threadably engage with the threaded hole 107g to enable to adjust the contact stroke to the flat surface 61c of the clutch lever 61. The rotating member also has an irregularly shaped hole portion 107b symmetrically having parallel surfaces 107a which match the notches 66b of the sub tube member 66, and a hole portion 107k through which a 2-mm screw 401 threadably engages with a threaded hole portion 126a of the annular member 126 so that the rotating member 107 can be fixed at the distal end of the sub tube member 66. A lid member 106 made of a resin to protect the wiring line 105 is snapped on the rotating member 107.

A protrusion 101d of the sensor actuator 101 is located above an upper surface 61d of the clutch lever 61 so that the actuator portion 101a retracts upon manually operating the clutch lever.

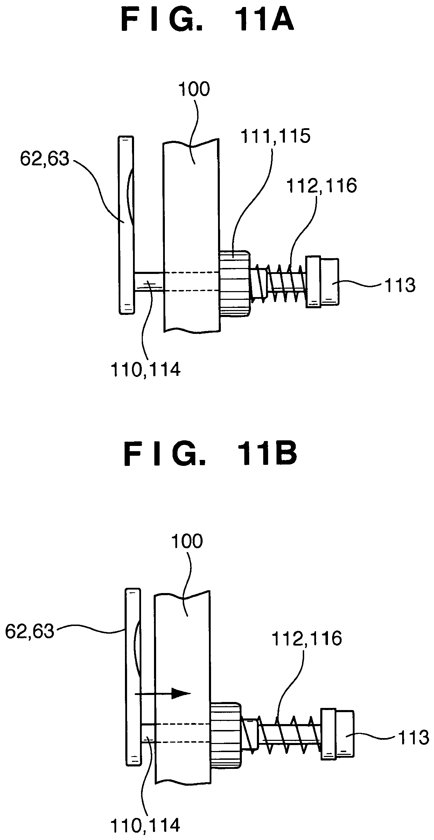

With the above-described arrangement, before manually operating the clutch lever 61, the pair of grip members 62 and 63 are in tight contact with the slider main body 100 because the helical compression springs 112 and 116 act to move and bias the shaft bodies 110 and 114 in the direction of an arrow, as shown in Fig. 11B. When the user manually operates the clutch lever 61 to unmesh, the helical compression springs are compressed between the sector gears 111 and 115 and the inclined member 113 so as to separate the pair of grip members 62 and 63 from the slider main body 100.

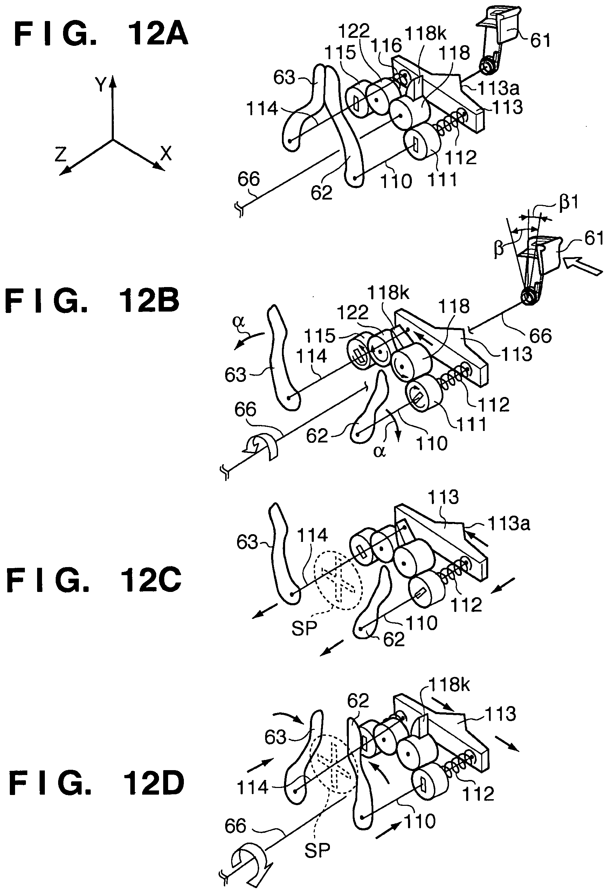

Figs. 12A to 12D are skeleton diagrams for explaining the operation of the grip moving mechanism. In Figs. 12A to 12D, the same reference numerals as those already described above denote the same components or parts, and a description thereof will not be repeated. Referring to Fig. 12A, before operating the clutch lever 61, the pair of left and right grip members 62 and 63 are closed as the rotary motion of the torsion spring 121 is transmitted to the intermediate gear 122, the sector gear 115 meshed with the intermediate gear 122, the intermediate gear 118, and the sector gear 111 meshed with the intermediate gear 118.

When the user manually presses the operation surface of the clutch lever 61 to rotate it by β1 out of the range of a maximum angle β, the helical compression springs 112 and 116 are compressed between the sector gears 111 and 115 and the inclined member 113, as shown in Fig. 11A. The pair of grip members 62 and 63 are separated from the slider main body 100 while remaining closed. When the user then presses the operation surface 61a of the clutch lever 61 up to the maximum angle β, the second abutment surface 61g (see

Fig. 10) of the clutch lever 61 comes into contact with the protrusion 118k of the intermediate gear 118 so as to rotate the intermediate gear 118 counterclockwise and the sector gear 111 clockwise. The intermediate gear 122 rotates clockwise, and the sector gear 115 rotates counterclockwise. Hence, the pair of left and right grip members 62 and 63 respectively rotate by the angle α and open (see Fig. 12B).

After that, the syringe plunger SP is set, as indicated by broken lines in Fig. 12C. Referring to Fig. 12D, when the operation of the clutch lever 61 is canceled, the grip members 62 and 63 do not close completely due to the radial size of the set syringe plunger SP. In addition, the sub tube member 66 rotates in the direction of double arrow in Fig. 12D and meshes with the threaded shaft body. The pair of grip members 62 and 63 move toward the slider main body 100 to bring the syringe plunger SP into tight contact with the slider main body 100, as shown in Fig. 11B. This allows to grip the syringe plunger SP in tight contact with the slider main body 100 without any gap.

As is apparent from the above description, the syringe pump 1 according to the embodiment is configured as follows.
(1) The slider assembly 6 which presses the syringe plunger SP of the set syringe S in the longitudinal direction (Z-axis direction) of the syringe S includes:
   - the grip members 62 and 63 which rotate, on the press surface to press the syringe plunger SP, between a closed position and an open position for gripping the syringe plunger SP and are biased in the closing direction and also toward the press surface, and
   - the clutch lever 61 which is arranged on a side surface of the slider assembly, biased to the OFF position, and rotates between the ON position and the OFF position.
(2) The sub tube member 66 having the slider assembly 6 attached to its distal end and meshed with the mesh mechanism 81 which functions as a conversion mechanism for converting the rotating operation upon driving the stepping motor 78 into the operation in the longitudinal direction (Z-axis direction)
   - moves in the longitudinal direction (Z-axis direction), when meshed with the mesh mechanism 81, upon driving the stepping motor 78 so as to move the slider assembly 6 in the longitudinal direction and press the syringe plunger SP, and
   - unmeshes from the mesh mechanism 81 upon rotating in the circumferential direction (direction of double arrow in Fig. 12B) so as to be manually movable in the longitudinal direction independently of drive of the stepping motor 78.
(3) The sub tube member 66 rotates in the circumferential direction (direction of double arrow in Fig. 12B) as the clutch lever 61 rotates.
(4) The inclined member 113, first sector gear 111, second sector gear 115, first intermediate gear 118, and second intermediate gear 122 are arranged in the slider assembly 6 as members to operate the grip members 62 and 63 in accordance with the rotation of the clutch lever 61.
(5) The inclined member 113 moves in the longitudinal direction when the clutch lever 61 rotates and comes into contact with the inclined member 113. Additionally, as the inclined member 113 moves in the longitudinal direction,
   - the shaft body 110 which can slide in the longitudinal direction relative to the first sector gear 111 and transmit the rotating force of the first sector gear 111 to the grip member 62 moves in the longitudinal direction against the biasing force toward the press surface. This allows to temporarily separate the grip member 62 from the press surface of the slider assembly 6 to grip the syringe plunger SP between the grip member and the press surface.
   - The shaft body 114 which can slide in the longitudinal direction relative to the second sector gear 115 and transmit the rotating force of the second sector gear 115 to the grip member 63 moves in the longitudinal direction against the biasing force toward the press surface. This allows to temporarily separate the grip member 63 from the press surface of the slider assembly 6 to grip the syringe plunger SP between the grip member and the press surface.
(6) The first intermediate gear 118 rotates when the clutch lever 61 rotates and comes into contact with the protrusion 118k of the first intermediate gear 118. Additionally, when the first intermediate gear 118 rotates,
   - the second intermediate gear 122 meshed with the first intermediate gear 118 rotates, and the second sector gear 115 meshed with the second intermediate gear 122 rotates so as to rotate the grip member 63 from the close position to the open position, and
   - the first sector gear meshed with the first intermediate gear 118 rotates so as to rotate the grip member 62 from the close position to the open position.

The above-described arrangement allows the user to easily grip the syringe plunger SP in tight contact with the slider assembly 6 independently of the diameter and thickness of the syringe plunger only by setting the syringe S, and in this state, moving the slider assembly 6 in the Z-axis direction up to the contact position to the syringe plunger SP while rotating the clutch lever 61 of the slider assembly 6 to the ON position.

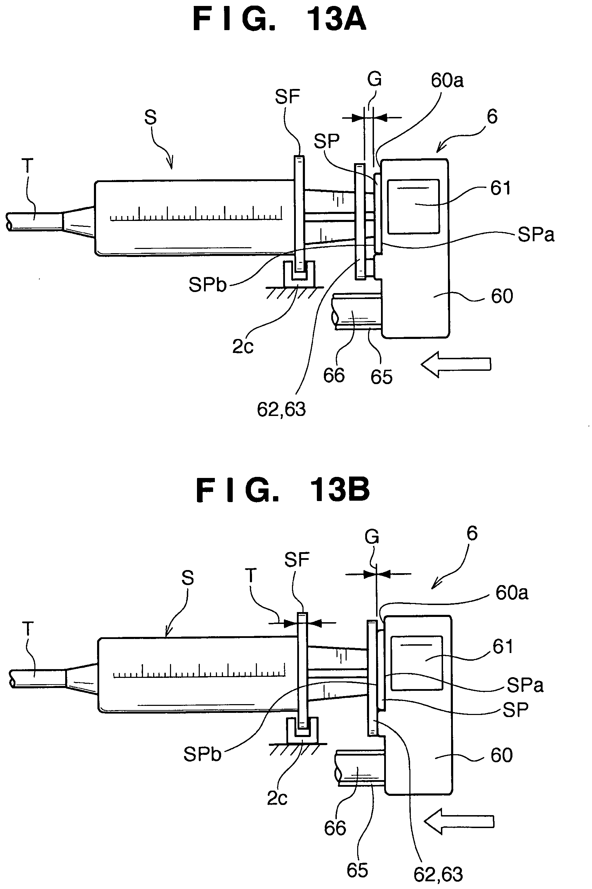

That is, a gap G formed when the syringe plunger SP comes into contact with a contact surface 60a of the cover 60, as shown in Fig. 13A, can be eliminated, as shown in Fig. 13B. This allows to absorb variations in the thickness between a surface SPa and a rear surface SPb of the syringe plunger SP.

As a result, it is possible to provide a syringe pump capable of holding the plungers of various types of syringes in tight contact with the slider assembly by a simple operation.

The present invention is not limited to the above embodiment and various changes and modifications can be made within the scope of the present invention. Therefore, to apprise the public of the scope of the present invention, the following claims are made.

## Claims

1. A syringe pump (1) including:
a syringe fixing portion (2b, 2c, 5) integrally formed on a pump main body (2) to fix a syringe main body (SB) of a syringe (S) filled with a drug;
a guide member (74, 80) arranged on the pump main body (2) so as to guide, along a longitudinal direction of the syringe (S), a main tube member (65) running from the pump main body (2) along the longitudinal direction of the syringe and a sub tube member (66) rotatably provided in the main tube member (65); and
a mesh mechanism (81) provided at one end of the sub tube member (66) to unmesh, by manually operating a clutch lever (61) biased to a standby position, the sub tube member (66) which is driven by a motor (78) and biased so as to mesh with a conversion mechanism (81) having a threaded shaft body (75) with a thread continually formed in the longitudinal direction for converting a rotating operation upon driving the motor (78) into an operation in the longitudinal direction so as to move a slider assembly (6) in the longitudinal direction, wherein:
said slider assembly (6) is provided at the other end of each of the main tube member (65) and the sub tube member (66) and pivotally supporting a pair of grip members (62, 63) which grip a syringe plunger (SP) upon operating the clutch lever,
said clutch lever (61) rotates between an open position for enabling an operation of gripping the syringe plunger and a fixed position for enabling the gripped state of the syringe plunger and is biased toward the fixed position,
said slider assembly (6) comprises a grip moving mechanism which brings the syringe plunger (SP) into tight contact with said slider assembly (6) by opening the pair of grip members (62, 63) upon manually operating the clutch lever (61), after setting the syringe plunger (SP), closing the pair of grip members (62, 63) upon canceling the manual operation, and moving the pair of grip members (62, 63) toward said slider assembly (6),
the clutch lever (61) is provided to be rotatable about the sub tube member (66) and rotates a rotating member (107) fixed at the other end of the sub tube member (66) into the unmeshed state upon the manual operation, and
said slider assembly (6) comprises a slider main body (100) fixed at the other end of the main tube member (65); **characterized in that**:
said grip moving mechanism comprises:
a first shaft body (110) and a second shaft body (114) each of which fixes a corresponding one of the pair of grip members (62, 63) at one end and is provided to be rotatable in a pivotal support hole portion (100b, 100d) formed in said slider main body (100) and movable in the longitudinal direction of the pivotal support hole portion (100b, 100d);
a first sector gear (111) provided to be slidable in the longitudinal direction of said first shaft body (110) and rotatable integrally;
a second sector gear (115) provided to be slidable in the longitudinal direction of said second shaft body (114) and rotatable integrally;
a first intermediate gear (118) and a second intermediate gear (122) which are rotatably pivotally supported by said slider main body (100) between said first sector gear (111) and said second sector gear (115) and mesh with said first sector gear (111) and said second sector gear (115), respectively;
a torsion spring (121) provided on one of said first intermediate gear (118) and said second intermediate gear (122) to move and bias the pair of grip members (62, 63) to the close state;
an inclined member (113) provided to connect the other end of said first shaft body (110) to the other end of said second shaft body (114) and having an inclined surface (113a) which comes into contact with a first abutment surface (61h) formed on the clutch lever (61) upon the manual operation;
a protrusion (118g) running from said first intermediate gear (118) in a radial direction so as to come into contact with the first abutment surface (61h) of the clutch lever (61) and then a second abutment surface upon the manual operation; and
helical compression springs (112, 116) provided between said inclined member (113) and said first sector gear (111) and said second sector gear (115) so as to move and bias the pair of grip members (62, 63) toward said slider main body (100) .

2. The syringe pump (1) according to claim 1, **characterized in that** the clutch lever (61) has, for the manual operation, an operation surface (61a) formed on a front side of the pump main body (2).

3. The syringe pump (1) according to claim 1 or 2, **characterized by** further comprising a cover (60) which covers said slider main body (100),
wherein the syringe plunger (SP) is brought into tight contact with said cover (60) of said slider assembly (6).

4. The syringe pump (1) according to claim 3, **characterized in that** the pair of grip members (62, 63) have shaped portions which grip an edge of the syringe plunger (SP) whose diameter changes depending on a syringe volume, and move the syringe plunger (SP) toward said cover (60).

5. A syringe pump (1) which infuses a drug in a syringe (S), the syringe pump (1) comprising:
a slider assembly (6) which presses a plunger (SP) of the set syringe (S) in a longitudinal direction of the syringe (S); and
a tube member (66) to which said slider assembly (6) is attached and which meshes with a conversion mechanism (81) for converting a rotating operation upon driving a motor into an operation in the longitudinal direction so as to move said slider assembly (6) in the longitudinal direction,
wherein said slider assembly (6) further comprises:
a pair of grip members (62, 63) which rotate, on a press surface to press the syringe plunger (SP), between a closed position and an open position for gripping the syringe plunger (SP) and are biased in a closing direction and also toward the press surface;
a clutch lever (61) which rotates between an operation position for enabling an operation of gripping the plunger (SP) and a fixed position for enabling the gripped state of the plunger (SP) and is biased toward the fixed position; **characterized in that** the syringe pump further comprises:
a rotation transmission member (111, 115, 118, 122) which transmits a rotating force upon rotating said clutch lever (61) to the operation position;
an inclined member (113) which moves in the longitudinal direction as said clutch lever (61) rotates to the operation position; and
shaft body (110, 114)which is slidable in the longitudinal direction relative to said rotation transmission member (111, 115, 118, 122) and able to transmit the rotating force to said grip members (62, 63), wherein:
as said clutch lever (61) rotates to the operation position, said tube member (66) rotates to cancel mesh with the conversion mechanism (81), and said slider assembly (6) becomes manually movable in the longitudinal direction independently of drive of the motor,
as said clutch lever (61) rotates to the operation position, said inclined member (113) moves in the longitudinal direction so as to slide said shaft body (110, 122) in the longitudinal direction and separate said pair of grip members (62, 63) from the press surface, and
as said clutch lever (61) rotates to the operation position, said shaft body (110, 122) transmits the rotating force transmitted by said rotation transmission member (111, 115, 118, 122) to said grip members (62, 63) so as to rotate, to the open position, said pair of grip members (62, 63) biased to the closed position.

## Patentansprüche

1. Spritzenpumpe (1), umfassend:
einen Spritzenfestlegungsabschnitt (2b, 2c, 5), der auf einem Pumpenhauptkörper (2) einstückig ausgebildet ist, um einen Spritzenhauptkörper (SB) einer Spritze (S), die mit einem Arzneimittel gefüllt ist, zu festzulegen;
ein Führungsteil (74, 80), das auf dem Pumpenhauptkörper (2) derart angeordnet ist, dass es entlang einer Längsrichtung der Spritze (S) ein Hauptrohrelement (65), das von dem Pumpenhauptkörper (2) entlang der Längsrichtung der Spritze verläuft, und ein Nebenrohrelement (66), das drehbar in dem Hauptrohrelement (65) vorgesehen ist, führt; und
einen Kämmungseingriffsmechanismus (81), der an einem Ende des Nebenrohrelements (66) vorgesehen ist, um durch manuelles Betätigen eines in eine Bereitschaftsposition vorgespannten Kupplungshebels (61) das Nebenrohrelement (66) außer Kämmungseingriff zu bringen, das durch einen Motor (78) angesteuert wird und derart vorgespannt ist, dass es mit einem Umwandlungsmechanismus (81) in kämmendem Eingriff ist, der einen Gewinde-Schaftkörper (75) mit einem in der Längsrichtung kontinuierlich ausgebildeten Gewinde aufweist, um eine Drehbetätigung bei Ansteuerung des Motors (78) in eine Betätigung in die Längsrichtung umzuwandeln, um so eine Schieberanordnung (6) in die Längsrichtung zu bewegen,
wobei die Schieberanordnung (6) am anderen Ende von jedem des Hauptrohrelements (65) und des Nebenrohrelements (66) vorgesehen ist und schwenkbar ein Paar von Greifelementen (62, 63) lagert, die bei Betätigen des Kupplungshebels einen Spritzenkolben (SP) ergreifen,
wobei sich der Kupplungshebel (61) zwischen einer offenen Position zum Ermöglichen einer Betätigung des Ergreifens des Spritzenkolbens und einer festgelegten Position zum Ermöglichen des ergriffenen Zustands des Spritzenkolbens dreht, und in die festgelegte Position hin vorgespannt ist,
wobei die Schieberanordnung (6) einen Greifbewegungsmechanismus umfasst, der den Spritzenkolben (SP) in engen Kontakt mit der Schieberanordnung (6) bringt, und zwar durch Öffnen des Greifelemente (62, 63) -Paars bei manueller Betätigung des Kupplungshebels (61) nach Einstellen des Spritzenkolbens (SP), Schließen des Greifelemente (62, 63) -Paars bei Beenden der manuellen Betätigung, und Bewegen des Greifelemente (62, 63) -Paars zur Schieberanordnung (6) hin,
wobei der Kupplungshebel (61) so vorgesehen ist, dass er um das Nebenrohrelement (66) drehbar ist, und ein Drehelement (107), das am anderen Ende des Nebenrohrelements (66) festgelegt ist, bei manueller Betätigung in den ausgerückten Zustand dreht, und
wobei die Schieberanordnung (6) einen Schieberhauptkörper (100) umfasst, der am anderen Ende des Hauptrohrelements (65) festgelegt ist;
**dadurch gekennzeichnet,**
**dass** der Greifbewegungsmechanismus Folgendes umfasst:
einen ersten Schaftkörper (110) und einen zweiten Schaftkörper (114), die jeweils ein entsprechendes von dem Greifelemente (62, 63) -Paar an einem Ende festlegen, und so vorgesehen sind, dass sie in einem Schwenklagerlochabschnitt (100b, 100d), der im Schieberhauptkörper (100) ausgebildet ist, drehbar und in die Längsrichtung des Schwenklagerlochabschnitts (100b, 100d) bewegbar sind;
ein erstes Zahnsegment (111), das vorgesehen ist, um in die Längsrichtung des ersten Schaftkörpers (110) verschiebbar und einstückig drehbar zu sein;
ein zweites Zahnsegment (115), das vorgesehen ist, um in die Längsrichtung des zweiten Schaftkörpers (114) verschiebbar und einstückig drehbar zu sein;
ein erstes Zwischenrad (118) und ein zweites Zwischenrad (122), die von dem Schieberhauptkörper (100) zwischen dem ersten Zahnsegment (111) und dem zweiten Zahnsegment (115) drehbar schwenkbar gelagert sind, und mit dem ersten Zahnsegment (111) beziehungsweise dem zweiten Zahnsegment (115) in kämmendem Eingriff sind;
eine Torsionsfeder (121), die auf einem des ersten Zwischenrades (118) und des zweiten Zwischenrades (122) vorgesehen ist, um das Greifelemente (62, 63) - Paar in den geschlossenen Zustand zu bewegen und vorzuspannen;
ein geneigtes Element (113), das vorgesehen ist, um das andere Ende des ersten Schaftkörpers (110) mit dem anderen Ende des zweiten Schaftkörpers (114) zu verbinden, und eine geneigte Oberfläche (113a) aufweist, die bei der manuellen Betätigung in Kontakt mit einer ersten Anlagefläche (61h) kommt, die auf dem Kupplungshebel (61) ausgebildet ist;
einen Vorsprung (118g), der von dem ersten Zwischenrad (118) in einer radialen Richtung verläuft, um so bei der manuellen Betätigung in Kontakt mit der erste Anlagefläche (61h) des Kupplungshebels und dann mit einer zweiten Anlagefläche zu kommen; und
spiralförmige Kompressionsringe (112, 116), die zwischen dem geneigten Element (113) und dem ersten Zahnsegment (111) und dem zweiten Zahnsegment (115) vorgesehen sind, um so das Greifelemente (62, 63) -Paar zum Schieberhauptkörper (100) hin zu bewegen und vorzuspannen.

2. Spritzenpumpe (1) gemäß Anspruch 1,
**dadurch gekennzeichnet,**
**dass** der Kupplungshebel (61) für die manuelle Betätigung eine Betätigungsfläche (61a) aufweist, die auf einer Stirnseite des Pumpenhauptkörpers (2) ausgebildet ist.

3. Spritzenpumpe (1) gemäß Anspruch 1 oder 2,
**gekennzeichnet durch** ein weiteres Umfassen einer Abdeckung (60), die den Schieberhauptkörper (100) abdeckt,
wobei der Spritzenkolben (SP) in engen Kontakt mit der Abdeckung (60) der Schieberanordnung (6) gebracht ist.

4. Spritzenpumpe (1) gemäß Anspruch 3,
**dadurch gekennzeichnet, dass** das Greifelemente (62, 63) -Paar geformte Abschnitte aufweist, die eine Kante des Spritzenkolbens (SP) ergreifen, dessen Durchmesser sich in Abhängigkeit von einem Spritzenvolumen ändert, und den Spritzenkolben (SP) zur Abdeckung (60) hin bewegen.

5. Spritzenpumpe (1), die in eine Spritze (S) ein Arzneimittel infundiert, wobei die Spritzenpumpe (1) Folgendes umfasst:
eine Schieberanordnung (6), die einen Kolben (SP) der eingestellten Spritze (S) in eine Längsrichtung der Spritze (S) drückt; und
ein Rohrelement (66), an dem die Schieberanordnung (6) befestigt ist und das mit einem Umwandlungsmechanismus (81) in kämmendem Eingriff ist, um eine Drehbetätigung bei Ansteuerung eines Motors in eine Betätigung in die Längsrichtung umzuwandeln, um so die Schieberanordnung (6) in die Längsrichtung zu bewegen,
wobei die Schieberanordnung (6) ferner Folgendes umfasst:
ein Paar von Greifelementen (62, 63), die sich auf einer Pressfläche drehen, um auf den Spritzenkolben (SP) zwischen einer geschlossenen Position und einer offenen Position zum Ergreifen des Spritzenkolbens (SP) zu drücken, und in eine Schließposition als auch zur Pressfläche hin vorgespannt sind;
einen Kupplungshebel (61), der sich zwischen einer Betätigungsposition zum Ermöglichen einer Betätigung des Ergreifens des Kolbens (SP) und einer festgelegten Position zum Ermöglichen des ergriffenen Zustands des Kolbens dreht, und zur festgelegten Position hin vorgespannt ist;
**dadurch gekennzeichnet, dass** der Spritzenkolben ferner Folgendes umfasst:
ein Drehübertragungselement (111, 115, 118, 122), das eine Drehkraft bei Drehen des Kupplungshebels (61) in die Betätigungsposition überträgt;
ein geneigtes Element (113), das sich bei Drehen des Kupplungshebels (61) in die Betätigungsposition in die Längsrichtung bewegt; und
einen Schaftkörper (110, 114), der relativ zum Drehübertragungselement (111, 115, 118, 122) in die Längsrichtung verschiebbar ist, und geeignet ist, die Drehkraft auf die Greifelemente (62, 63) zu übertragen,
wobei dann, wenn sich der Kupplungshebel (61) in die Betätigungsposition dreht, das Rohrelement (66) sich dreht, um den kämmenden Eingriff mit dem Umwandlungsmechanismus (81) zu beenden, und die Schieberanordnung (6) in die Längsrichtung manuell bewegbar wird, und zwar unabhängig vom Ansteuern des Motors,
wobei dann, wenn sich der Kupplungshebel (61) in die Betätigungsposition dreht, das geneigte Element (113) sich in die Längsrichtung bewegt, um so den Schaftkörper (110, 122) in die Längsrichtung zu verschieben, und das Greifelemente (62, 63) -Paar von der Pressfläche zu trennen, und
wobei dann, wenn sich der Kupplungshebel (61) in die Betätigungsposition dreht, der Schaftkörper (110, 122) die von dem Drehübertragungselement (111, 115, 118, 122) übertragene Drehkraft auf die Greifelemente (62, 63) überträgt, um sich so in die offene Position zu drehen, wobei das Greifelemente (62, 63) -Paar in die geschlossene Position vorgespannt ist.

## Revendications

1. Pompe à seringue (1) comprenant :
une partie de fixation de seringue (2b, 2c, 5) formée d'un seul tenant sur un corps principal de pompe (2) pour fixer un corps principal de seringue (SB) d'une seringue (S) remplie d'un médicament ;
un élément de guidage (74, 80) agencé sur le corps principal de pompe (2) de manière à guider, le long d'une direction longitudinale de la seringue (S), un élément de tube principal (65) s'étendant à partir du corps principal de pompe (2) le long de la direction longitudinale de la seringue et un sous-élément de tube (66) disposé en rotation dans l'élément de tube principal (65) ; et
un mécanisme d'engrènement (81) disposé au niveau d'une extrémité du sous-élément de tube (66) pour désengrener, par l'actionnement manuel d'un levier d'embrayage (61) sollicité vers une position de veille, le sous-élément de tube (66) qui est entraîné par un moteur (78) et sollicité de manière à s'engrener avec un mécanisme de conversion (81) comprenant un corps d'arbre fileté (75) avec un filetage formé en continu dans la direction longitudinale pour convertir une opération de rotation lors de l'entraînement du moteur (78) en une opération dans la direction longitudinale de manière à déplacer un ensemble coulisse (6) dans la direction longitudinale, dans laquelle :
ledit ensemble coulisse (6) est disposé au niveau de l'autre extrémité de chacun de l'élément de tube principal (65) et du sous-élément de tube (66) et supportant de façon pivotante une paire d'éléments de préhension (62, 63) qui saisissent un piston de seringue (SP) lors de l'actionnement du levier d'embrayage,
ledit levier d'embrayage (61) tourne entre une position ouverte pour permettre une opération de préhension du piston de seringue et une position fixe pour permettre l'état de préhension du piston de seringue et est sollicité en direction de la position fixe,
ledit ensemble coulisse (6) comprend un mécanisme de déplacement de préhension qui amène l'ensemble coulisse (6) en contact étroit avec ledit ensemble coulisse (6) par l'ouverture de la paire d'éléments de préhension (62, 63) lors de l'actionnement manuel du levier d'embrayage (61), après le montage du piston de seringue (SP), la fermeture de la paire d'éléments de préhension (62, 63) lors de l'annulation de l'actionnement manuel, et le déplacement de la paire d'éléments de préhension (62, 63) en direction dudit ensemble coulisse (6),
le levier d'embrayage (61) est disposé de manière à être rotatif autour du sous-élément de tube (66) et entraîne un élément de rotation (107) fixé au niveau de l'autre extrémité du sous-élément de tube (66) dans l'état non engrené lors de l'actionnement manuel, et
ledit ensemble coulisse (6) comprend un corps principal de coulisse (100) fixé au niveau de l'autre extrémité de l'élément de tube principal (65) ;
**caractérisée en ce que** :
ledit mécanisme de déplacement de préhension comprend :
un premier corps d'arbre (110) et un second corps d'arbre (114) dont chacun fixe un élément de préhension correspondant de la paire d'éléments de préhension (62, 63) au niveau d'une extrémité et est disposé de manière à être rotatif dans une partie de trou de support pivotante (100b, 100d) formée dans ledit corps principal de coulisse (100) et mobile dans la direction longitudinale de la partie de trou de support pivotante (100b, 100d) ;
un premier secteur denté (111) disposé de manière à être coulissant dans la direction longitudinale dudit premier corps d'arbre (110) et rotatif d'un seul tenant ;
un second secteur denté (115) disposé de manière à être coulissant dans la direction longitudinale dudit second corps d'arbre (114) et rotatif d'un seul tenant ;
un premier engrenage intermédiaire (118) et un second engrenage intermédiaire (122) qui sont supportés en rotation de façon pivotante par ledit corps principal de coulisse (100) entre ledit premier secteur denté (111) et ledit second secteur denté (115) et s'engrènent avec ledit premier secteur denté (111) et ledit second secteur denté (115), respectivement ;
un ressort de torsion (121) disposé sur l'un desdits premier engrenage intermédiaire (118) et second engrenage intermédiaire (122) pour déplacer et solliciter la paire d'éléments de préhension (62, 63) vers l'état fermé ;
un élément incliné (113) disposé de manière à raccorder l'autre extrémité dudit premier corps d'arbre (110) à l'autre extrémité dudit second corps d'arbre (114) et comprenant une surface inclinée (113a) qui vient en contact avec une première surface de butée (61h) formée sur le levier d'embrayage (61) lors de l'actionnement manuel ;
une saillie (118g) s'étendant à partir dudit premier engrenage intermédiaire (118) dans une direction radiale de manière à venir en contact avec la première surface de butée (61h) du levier d'embrayage (61) puis une seconde surface de butée lors de l'actionnement manuel ; et
des ressorts de compression hélicoïdaux (112, 116) disposés entre ledit élément incliné (113) et ledit premier secteur denté (111) et ledit second secteur denté (115) de manière à déplacer et solliciter la paire d'éléments de préhension (62, 63) en direction dudit corps principal de coulisse (100).

2. Pompe à seringue (1) selon la revendication 1, **caractérisée en ce que** le levier d'embrayage (61) comprend, pour l'actionnement manuel, une surface d'actionnement (61a) formée sur un côté avant du corps principal de pompe (2) .

3. Pompe à seringue (1) selon la revendication 1 ou 2, **caractérisée en ce qu'**elle comprend en outre un couvercle (60) qui recouvre ledit corps principal de coulisse (100),
dans laquelle le piston de seringue (SP) est amené en contact étroit avec ledit couvercle (60) dudit ensemble coulisse (6).

4. Pompe à seringue (1) selon la revendication 3, **caractérisée en ce que** la paire d'éléments de préhension (62, 63) comprend des parties formées qui saisissent un bord du piston de seringue (SP) dont le diamètre change en fonction d'un volume de seringue, et déplacent le piston de seringue (SP) en direction dudit couvercle (60).

5. Pompe à seringue (1) qui infuse un médicament dans une seringue (S), la pompe à seringue (1) comprenant :
un ensemble coulisse (6) qui presse un piston (SP) de la seringue montée (S) dans une direction longitudinale de la seringue (S) ; et
un élément de tube (66) auquel ledit ensemble coulisse (6) est attaché et qui s'engrène avec un mécanisme de conversion (81) pour convertir une opération de rotation lors de l'entraînement d'un moteur en une opération dans la direction longitudinale de manière à déplacer ledit ensemble coulisse (6) dans la direction longitudinale,
dans laquelle ledit ensemble coulisse (6) comprend en outre :
une paire d'éléments de préhension (62, 63) qui tournent, sur une surface de pression pour presser le piston de seringue (SP), entre une position fermée et une position ouverte pour saisir le piston de seringue (SP) et sont sollicités dans une direction de fermeture et également en direction de la surface de pression ;
un levier d'embrayage (61) qui tourne entre une position d'actionnement pour permettre une opération de préhension du piston (SP) et une position fixe pour permettre l'état de préhension du piston (SP) et est sollicité en direction de la position fixe ; **caractérisée en ce que** la pompe à seringue comprend en outre :
un élément de transmission de rotation (111, 115, 118, 122) qui transmet une force de rotation lors de la rotation dudit levier d'embrayage (61) vers la position d'actionnement ;
un élément incliné (113) qui se déplace dans la direction longitudinale lorsque ledit levier d'embrayage (61) tourne vers la position d'actionnement ; et
un corps d'arbre (110, 114) qui est coulissant dans la direction longitudinale par rapport audit élément de transmission de rotation (111, 115, 118, 122) et apte à transmettre la force de rotation auxdits éléments de préhension (62, 63), dans laquelle :
lorsque ledit levier d'embrayage (61) tourne vers la position d'actionnement, ledit élément de tube (66) tourne pour annuler l'engrènement avec le mécanisme de conversion (81), et ledit ensemble coulisse (6) devient manuellement mobile dans la direction longitudinale indépendamment de l'entraînement du moteur,
lorsque ledit levier d'embrayage (61) tourne vers la position d'actionnement, ledit élément incliné (113) se déplace dans la direction longitudinale de manière à faire coulisser ledit corps d'arbre (110, 122) dans la direction longitudinale et séparer ladite paire d'éléments de préhension (62, 63) de la surface de pression, et
lorsque ledit levier d'embrayage (61) tourne vers la position d'actionnement, ledit corps d'arbre (110, 122) transmet la force de rotation transmise par ledit élément de transmission de rotation (111, 115, 118, 122) auxdits éléments de préhension (62, 63) de manière à tourner, vers la position ouverte, ladite paire d'éléments de préhension (62, 63) sollicitée vers la position fermée.
